Europäisches Patentamt

European Patent Office

Office européen des brevets

(19)

(11) Veröffentlichungsnummer: **0 289 853**
**A1**

## (12) EUROPÄISCHE PATENTANMELDUNG

(21) Anmeldenummer: **88106239.2**

(22) Anmeldetag: **20.04.88**

(51) Int. Cl.⁴ **A61F 2/04 , A61M 25/00**

(30) Priorität: **05.05.87 DE 3714839**

(43) Veröffentlichungstag der Anmeldung:
**09.11.88 Patentblatt 88/45**

(84) Benannte Vertragsstaaten:
**AT BE CH ES FR GB GR IT LI NL SE**

(71) Anmelder: **UROMED KURT DREWS GMBH**
**Gewerbering 8**
**D-2000 Oststeinbek(DE)**

(72) Erfinder: **Drews, Kurt**
**Zum Osterstein 18**
**D-2000 Oststeinbek(DE)**

(74) Vertreter: **Schaefer, Konrad**
**Gehölzweg 20**
**D-2000 Hamburg 70(DE)**

(54) **Ureterschiene mit Vorschubschlauch und Mandrin.**

(57) Eine Ureterschiene (1) ist zum Zwecke der Verlegung mit einem verlängernden Vorschubschlauch (2) und einem innenliegenden Mandrin (3) durch Einklemmen eines Ansatzes (4) an der Verbindungsstelle zwischen Ureterschiene (1) und Vorschubschlauch (2) verbunden.

EP 0 289 853 A1

## Ureterschiene mit Vorschubschlauch und Mandrin.

Die Erfindung betrifft eine Ureterschiene der im Oberbegriff des Anspruches 1 genannten Art.

Ureterschienen dienen bei die Urindurchgängigkeit hindernden Erkrankngen des die Niere mit der Blase verbindenden Ureters zur Sicherstellung des Urinabganges aus der Niere. Sie werden mit einem durch die Harnröhre in die Blase eingeführten Endoskop von der Blase her in den Ureter bis zur Niere vorgeschoben und sind in der Regel so ausgebildet, daß sie mit freien Enden in der Niere und in der Blase liegen, die gebogen ausgeführt sind, um das Verrutschen zu verhindern. An beiden Enden sind Öffnungen vorgesehen, die den Urindurchfluß sichern. Ureterschienen sind als weiche Schläuche von wenigen Millimetern Durchmesser ausgebildet.

Zum Einführen werden diese weichen Schläuche mit einem eingelegten weniger flexiblen Mandrin (Draht bzw. Spiraldrahtschlauch oder Kombination aus beiden) so weit versteift, daß ein sicheres Vorschieben ohne Abknickgefahr auch durch Verengungen des Ureters möglich ist.

Probleme entstehen dabei dann, wenn an Engstellen des Ureters ein mehrfaches Vor-und Zurückschieben notwendig ist, um einen geeigneten Durchgang zu suchen.

Da die Schiene im wesentlichen in ihrer Länge nur von der Niere bis zur Blase reicht, also nicht durch das zum Einführen dienende Endoskop bis nach draußen, muß stets mit einem Vorschubschlauch gearbeitet werden, der ebenfalls auf dem Mandrin sitzt und das Vorschieben der Schiene ermöglicht. Die erwähnten Probleme entstehen dann, wenn außerhalb des Endoskopes zurückgezogen werden soll. Weder ein Zurückziehen am Vorschubschlauch noch ein Zurückziehen am Mandrin kann das Zurückziehen der unter Umständen fest sitzenden Schiene bewirken. Nach älterem Stand der Technik kann die Schiene also nur vorgeschoben und nicht zurückgezogen werden.

Aus dem DE-GM 86 14 013.2 ist eine Konstruktion bekannt, bei der der Vorschubschlauch mit der Ureterschiene über eine Perforation verbunden ist. Diese erlaubt beim Manipulieren während des Einführens der Ureterschiene durch Verbindung mit dem Vorschubschlauch das Vor-und Zurückziehen. Nach korrekter Verlegung der Ureterschiene muß die Perforation getrennt werden. Dazu wird am Vorschubschlauch und am Mandrin gegenläufig gezogen, wobei sich der Mandrin an der distalen Spitze der Ureterschiene, die geschlossen sein muß, abstützt. Es wird so stark gezogen, daß die Perforation zerreißt.

Aus der DE-OS 33 39 179 ist eine ähnliche Konstruktion bekannt, bei der die Ureterschiene distal auch offen sein kann. Hier wird mit dem Endoskop, also beispielsweise einer geeigneten Zange, die Ureterschiene festgehalten und dann am Vorschubschlauch bis zum Zerreißen der Perforation gezogen.

Diese Konstruktionen weisen aber eine Reihe von Nachteilen auf. So ist es schwierig, die Perforation so genau zu fertigen, daß sie beim Manipulieren hält, zum Zerreißen aber schwach genug ist. Es kommt aufgrund von Fertigungsungenauigkeiten häufig vor, daß die Perforation nicht zerrissen werden kann, ohne zu große, Verletzungsgefahren mit sich bringende Kräfte aufbringen zu müssen. Weiterhin sind am in der Blase liegenden proximalen Ende der Ureterschiene unter Umständen die Unebenheiten der Perforationsstelle störend und können beim Anliegen an der Blasenwand zu Irritationen führen. Außerdem erfordern die bekannten Vorrichtungen entweder eine Zange zum Halten der Ureterschiene oder ein geschlossenes Ende der Ureterschiene, an dem sich der Mandrin abstützt. Es ergeben sich also apparative bzw. konstruktive Einschränkungen, die unter Umständen störend sind.

Bei beiden genannten bekannten Konstruktionen besteht eine weitere apparative Beschränkung darin, daß beim Manipulieren zwar die Ureterschiene und der Vorschubschlauch verbunden sind, der Mandrin aber in seiner Position gehalten werden muß, um ein Herausrutschen zu verhindern. Zu diesem Zweck ist in der Regel am proximalen Ende des Vorschubschlauches dieser mit dem Mandrin durch eine Klemme zu verbinden. Diese erfordert zusätzliche Handhabungen oeim Anlegen und Lösen.

Die Aufgabe der vorliegenden Erfindung besteht daher darin, eine Ureterschiene der eingangs genannten Art zu schaffen, die sicher und einfach beim Manipulieren vor-und zurückgeschoben werden kann, ohne daß sich der Vorschubschlauch von der Ureterschiene löst, bei der anschließend das Lösen der Verbindung einfach und bequem möglich ist und die keine der apparativen und konstruktiven Beschränkungen des Standes der Technik aufweist.

Diese Aufgabe wird erfindungsgemäß mit den Merkmalen des Kennzeichnungsteiles des Anspruches 1 gelöst.

Nach der Erfindung wird an der Übergangsstelle vom Vorschubschlauch zur Ureterschiene eine Klemmverbindung hergestellt, mit der die Ureterschiene, der Vorschubschlauch und der Mandrin aneinander verklemmt werden. Dazu ist entweder an der Ureterschiene oder am Vorschubschlauch ein Ansatz vorgesehen, der mit verringertem Querschnitt in das Schlauchinnere des jeweils anderen Teiles gesteckt wird und dort mit dem ebenfalls durch das Schlauchinnere verlegten Mandrin im Schlauch verklemmt wird. Auf diese Weise sind alle drei beteiligten Teile aneinander arretiert, und es läßt sich die somit gebildete Einheit von Ureterschiene, Vorschubschlauch und Mandrin auf einfachste Weise beim Einführen der Ureterschiene in den Ureter manipulieren. Eine zusätzliche Klemme zur Verbindung des Vorschubschlauches mit dem Mandrin ist nicht erforderlich. Zum Lösen der Klemmvorrichtung wird außerhalb des zur Verlegung dienenden Endoskops am Mandrin einerseits und am Vorschubschlauch andererseits angefaßt und der Mandrin herausgezogen. Dieser muß nach erfolgter Verlegung der Ureterschiene ohnehin entfernt werden, so daß sich hierdurch kein zusätzlicher Arbeitsgang ergibt. Dabei bleibt die Ureterschiene sicher liegen, da sie sich in Zugrichtung am Vorschubschlauch abstützt. Nachdem das distale Ende des Mandrins die Klemmstelle passiert hat, löst sich die Verklemmung, und der Vorschubschlauch kann herausgezogen werden. Die erfindungsgemäße Konstruktion ergibt den weiteren Vorteil, daß die Trennstelle zwischen Ureterschiene und Vorschubschlauch beliebig gelegt werden kann, beispielsweise auch im Ureter selbst liegen kann. Es können also auch Ureterschienen auf erfindungsgemäße Weise verlegt werden, die proximal kurz vor dem Ostium, also der Mündung des Ureters in die Blase, enden.

Weiterhin vorteilhaft sind die Merkmale des Anspruches 2 vorgesehen. Wenn der Ansatz am Vorschubschlauch vorgesehen ist, kann das proximale Ende der Ureterschiene frei von Ansätzen glatt, also ohne Störstellen ausgebildet sein.

Weiterhin vorteilhaft sind die Merkmale des Anspruches 3 vorgesehen. Der Ansatz kann als Schlauchanschnitt konstruktiv sehr einfach vorgesehen sein, beispielsweise als zugespitztes Schlauchende oder eckig gestanzt, wobei der Ansatz also als Verlängerung des Schlauches vorgesehen ist, bei dem ein größerer Umfangsteil des Schlauches weggeschnitten ist.

Schließlich sind erfahrungsgemäß vorteilhaft die Merkmale des Anspruches 4 vorgesehen. Wird der Ansatz mit aufgerauhter Oberfläche versehen, so läßt sich dadurch die Klemmwirkung beeinflussen, also eine Klemmkraft einstellen, die für die vorliegenden Zwecke ausreichend ist, dennoch ein relativ leichtes Durchziehen des Mandrins zum Lösen der Verbindung ermöglicht. Natürlich kann die Klemmkraft auch über die Länge des Ansatzes in gewünschter Weise gesteuert werden.

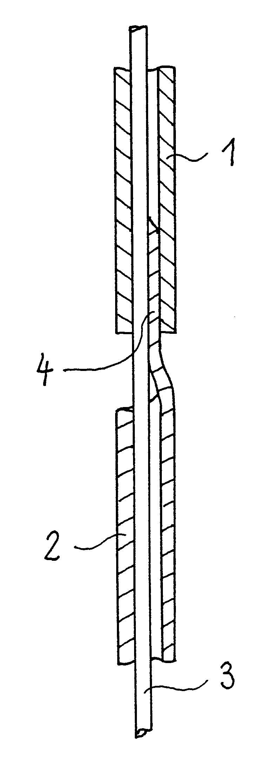

In der Zeichnung ist die Erfindung beispielsweise an Hand eines Achsschnittes durch die Verbindungsstelle zwischen Ureterschiene und Vorschubschlauch dargestellt.

Die Zeichnung zeigt das proximale, also zur Blase hin liegende Ende einer als weicher Schlauch geringen Durchmessers von beispielsweise 2 bis 3 mm ausgebildeten Ureterschiene 1 sowie das distale Ende eines Vorschubschlauches 2, der im dargestellten Ausführungsbeispiel aus demselben Material wie die Ureterschiene besteht. Durch Ureterschiene und Vorschubschlauch ist ein Mandrin 3 verlegt, der beispielsweise als Draht oder als Spiraldrahtschlauch oder Kombination von beiden in herkömmlicher Weise ausgebildet ist.

Am dargestellten distalen Ende des Vorschubschlauches 2 ist ein Ansatz 4 vorgesehen. Dieser ist im dargestellten Ausführungsbeispiel dadurch ausgebildet, daß das distale Ende des Vorschubschlauches 2 schräg zugespitzt angeschnitten ist, wobei sich der Ansatz 4 als Spitze ergibt, die aus einem Teil des Schlauchumfanges besteht.

Der Ansatz 4 kann wie im dargestellten Ausführungsbeispiel schräg zugespitzt ausgebildet sein oder mit konstantem Querschnitt, wobei er rechteckig in den vollständigen Vorschubschlauch 2 übergeht. Der Ansatz 4 kann aus gesondertem Material bestehen, das mit dem Vorschubschlauch 2 verbunden ist oder vorzugsweise, wie im dargestellten Ausführungsbeispiel, aus dem Schlauchmaterial des Vorschubschlauches 2.

Zur Herstellung der dargestellten Verbindung zwischen den Teilen 1, 2 und 3 wird zunächst der Ansatz 4 in den Innenraum der Ureterschiene 1 eingeführt und sodann der Mandrin 3 hindurchgeschoben. Der Querschnitt des Ansatzes 4 ist so gewählt, daß sich in der dargestellten Konfiguration zwischen Mandrin 3 und Innenwand der Ureterschiene 1 eine auf den Ansatz 4 einwirkende Klemmkraft ergibt. Alle drei Teile 1, 2 und 3 sind auf diese Weise miteinander verklemmt.

Mandrin 3 und Vorschubschlauch 2 ragen proximal aus dem bis in die Blase eingeführten Endoskop nach außen heraus. Es kann am Vorschubschlauch angefaßt und die verklemmte Einheit 1, 2, 3 vor-und zurückgeschoben werden, wobei das distale Ende der Ureterschiene 1 durch Verengungen des Ureters feinfühlig hindurchgefädelt werden kann, ohne daß sich die Verbindung zwischen Ureterschiene 1 und Vorschubschlauch 2 löst. Dabei bleibt der ebenfalls festgeklemmte Mandrin 3 in seiner Lage, so daß die Ureterschiene 1 sicher bis zu ihrem distalen Ende ausgesteift ist.

Nachdem die Ureterschiene 1 in ihre endgültige Lage gebracht worden ist, muß die Klemmverbindung gelöst werden. Dazu wird der Vorschubschlauch 2 festgehalten und der Mandrin herausgezogen. Wenn das distale Ende des Mandrins 3 die Klemmstelle, also den Ansatz 4, passiert hat, löst sich die Verklemmung, der Ansatz 4 wird also in der Ureterschiene 1 frei beweglich. Nun kann auch der Vorschubschlauch 2 herausgezogen werden.

Versuche haben gezeigt, daß das Lösen der Verbindung mit der erfindungsgemäßen Konstruktion sehr gut überwacht werden kann. Wird der Mandrin 3 durch die Klemmstelle zurückgezogen, so ergibt sich eine erhöhte Zugkraft, die sich - schlagartig verringert, wenn der Mandrin mit seinem distalen Ende durch die Klemmstelle hindurchgezogen ist. Daran merkt der Operateur auf äußerst ein fache und präzise Weise, wann die Verklemmung gelöst ist. Er kann sodann Mandrin 3 und Vorschubschlauch 2 gemeinsam herausziehen ohne jedes Risiko, die verlegte Ureterschiene 1 noch zu verschieben.

Die Klemmkraft kann über den Querschnitt und die Länge des Ansatzes 4 auf den gewünschten Wert eingestellt werden. Durch Aufrauhen der Oberflächen des Ansatzes 4 läßt sich die Klemmkraft weiterhin in gewünschter Weise beeinflussen.

Im dargestellten Ausführungsbeispiel ist der Ansatz 4 am Vorschubschlauch 2 vorgesehen. Ersichtlich läßt sich derselbe Klemmeffekt erzielen, wenn der Ansatz 4 am proximalen Ende der Ureterschiene 1 vorgesehen ist und in den Vorschubschlauch 2 hereinragend verklemmt wird. Die dargestellte Ausführungsform ist aber unter Umständen vorzuziehen, da sie ein glattes proximales Ende der Ureterschiene 1 ohne störende Spitzen ermöglicht.

## Ansprüche

1. Ureterschiene, die mit einem verlängernden Vorschubschlauch und einem durchgehenden, innenliegenden Mandrin zu einer vor-und zurückbewegbaren Einheit gekuppelt ist, dadurch gekennzeichnet, daß an der Übergangsstelle vom Vorschubschlauch (2) zur Ureterschiene (1) am angrenzenden Ende eines dieser Teile (2) ein Ansatz (4) vorgesehen ist, dessen Querschnitt so gewählt ist, daß der Ansatz (4) im anderen Teil (1) zwischen diesem und dem Mandrin (3) klemmend arretierbar ist.

2. Ureterschiene nach Anspruch 1, dadurch gekennzeichnet, daß der Ansatz (4) am Vorschubschlauch (2) vorgesehen ist.

3. Ureterschiene nach einem der vorhergehenden Ansprüche, dadurch gekennzeichnet, daß der Ansatz (4) als integraler Schlauchabschnitt ausgebildet ist.

4. Ureterschiene nach einem der vorhergehenden Ansprüche, dadurch gekennzeichnet, daß der Ansatz (4) mit aufgerauhter Oberfläche versehen ist.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,D | DE-U-8 614 013 (UROMED)<br>* Seite 4, Zeile 33 - Seite 7, Zeile 5; Figuren *<br>--- | 1 | A 61 F 2/04<br>A 61 M 25/00 |
| A | US-A-3 999 551 (SPITZ)<br>* Spalte 4, Zeilen 10-38; Figuren 8-10 *<br>--- | 1,2 | |
| A,D | DE-A-3 339 179 (BEIRING)<br>* Insgesamt *<br>--- | 1 | |
| A | US-A-4 195 624 (DOUGLAS)<br>--- | | |
| A | US-A-4 552 554 (GOULD)<br>----- | | |

**RECHERCHIERTE SACHGEBIETE (Int. Cl.4)**

A 61 F
A 61 M
A 61 B

Der vorliegende Recherchenbericht wurde für alle Patentansprüche erstellt

| Recherchenort | Abschlußdatum der Recherche | Prüfer |
|---|---|---|
| DEN HAAG | 04-08-1988 | KLEIN C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
................................................................................
& : Mitglied der gleichen Patentfamilie, übereinstimmendes Dokument

EPO FORM 1503 03.82 (P0403)